# EUROPEAN PATENT SPECIFICATION

(11) **EP 4 065 965 B1**
(45) Date of publication and mention of the grant of the patent: **22.10.2025**
(21) Application number: 20811333.2
(22) Date of filing: 25.11.2020
(51) Int. Cl.: G01N 21/84

(54) **METHOD OF PERFORMING AN ANALYTICAL MEASUREMENT**
VERFAHREN ZUR DURCHFÜHRUNG EINER ANALYTISCHEN MESSUNG
PROCÉDÉ DE RÉALISATION D'UNE MESURE ANALYTIQUE

(30) Priority: 26.11.2019 EP 19211519
(43) Date of publication of application: 05.10.2022
(73) Proprietor: F. Hoffmann-La Roche AG, 4070 Basel (CH); Roche Diabetes Care GmbH, 68305 Mannheim (DE)
(72) Inventor: ALPEROWITZ, Lukas, 80805 München (DE); BERG, Max, 68305 Mannheim (DE); SELLMAIR, Sebastian, 80805 München (DE)
(74) Representative: Meinel, Anne Julia
(86) International application number: PCT/EP2020/083384
(87) International publication number: WO 2021/105222

(56) References cited:
- EP-A1- 1 801 568
- WO-A1-2012/131386
- DE-A1- 102016 202 428
- US-A1- 2018 024 049

## Description

### Technical Field

The present application refers to a method of performing an analytical measurement based on a color formation reaction in an optical test strip by using a mobile device having a camera. The invention further relates to a computer program and a computer-readable storage medium with program means for executing the method according to the invention. Further, the invention refers to a mobile device and a kit for performing an analytical measurement. Methods, computer programs, mobile devices and kits according to the present invention may be used in medical diagnostics, in order to for example qualitatively or quantitatively detect one or more analytes in one or more body fluids. Other fields of application of the present invention, however, are feasible.

### Background art

In the field of medical diagnostics, in many cases, one or more analytes have to be detected in samples of a body fluid, such as blood, interstitial fluid, urine, saliva or other types of body fluids. Examples of analytes to be detected are glucose, triglycerides, lactate, cholesterol or other types of analytes typically present in these body fluids. According to the concentration and/or the presence of the analyte, an appropriate treatment may be chosen, if necessary. Without narrowing the scope, the invention specifically may be described with respect to blood glucose measurements. It shall be noted, however, that the present invention may also be used for other types of analytical measurements using test elements.

Generally, devices and methods known to the skilled person make use of test elements comprising one or more test chemicals, which, in presence of the analyte to be detected, are capable of performing one or more detectable detection reactions, such as optically detectable detection reactions. As an example, EP 0 821 234 A2 describes a diagnostic test carrier for the determination of an analyte from whole blood with the aid of a reagent system contained in the carrier and a method for the determination of an analyte from whole blood with the aid of the diagnostic test carrier. The diagnostic test carrier includes a color forming reagent. The test field has a sample application side to which the blood sample is delivered and a detection side where an optically detectable change occurs as a result of the reaction of the analyte with the reagent system. Furthermore, the test field is designed so that the erythrocytes contained in the sample do not reach the detection side. In addition, the test field comprises a transparent film and a first and a second superposed film layer applied thereto, wherein the first layer on the transparent film is substantially less light-scattering in the wet state than the overlying second layer.

With regard to the test chemicals comprised in test elements, reference may be made e.g. to J. Hoenes et al.: The Technology Behind Glucose Meters: Test Strips, Diabetes Technology & Therapeutics, Volume 10, Supplement 1, 2008, S-10 to S-26. Other types of test chemistry are possible and may be used for performing the present invention.

In analytical measurements, specifically analytical measurements based on color formation reactions, one technical challenge resides in the evaluation of the color change which is due to the detection reaction. Besides using dedicated analytical devices, such as handheld blood glucose meters, the use of generally available electronics such as smart phones and portable computers has become more and more popular over the recent years. WO 2012/131386 A1 discloses a testing apparatus for performing an assay, the testing apparatus comprising: a receptacle containing a reagent, the reagent being reactive to an applied test sample by developing a color or pattern variation; a portable device, e.g. a mobile phone or a laptop, comprising a processor and an image capture device, wherein the processor is configured to process data captured by the image capture device and output a test result for the applied test sample. Further, software Apps for use with a smart phone are available for download, such as the ACCU-CHEK^{®} SugarView App by Roche Diabetes Care GmbH, Germany, available under https://www.accu-chek-sugarview.com.

As opposed to laboratory measurements and measurements performed by using dedicated analytical measurement devices, when using mobile computing devices such as smart phones, various influences need to be taken into account. As an example, lighting conditions, positioning, vibrations or other more or less uncontrollable conditions are to be considered. In the field of technology of mobile computing devices, various technical approaches have been developed over the recent years in order to improve image recognition and in order to gain additional information regarding, for example, unknown geometrical parameters of the setup.

Thus, as an example, US 9691152 B1 discloses approaches for minimizing variations in the height of a camera of a computing device when estimating the distance to objects represented in image data captured by the camera. For example, a front-facing camera of a computing device can be used to capture a live camera view of a user. An application can analyze the image data to locate features of the user's face for purposes of aligning the user with the computing device. As the position and/orientation of the device changes with respect to the user, the image data can be analyzed to detect whether a location of a representation of a feature of the user aligns with the alignment element. Once the feature is aligned with the alignment element, a rear-facing camera or other camera can capture second image data of an object. The second image data can be analyzed to determine a geometric relationship between the rear-facing camera and the object, and the geometric relationship can be used to determine a distance to the object with respect to the computing device.

Further, US 9,934,612 B2 discloses a method for determining the pose of a camera with respect to an object of a real environment for use in authoring/augmented reality application that includes generating a first image by the camera capturing a real object of a real environment, generating first orientation data from at least one orientation sensor associated with the camera or from an algorithm which analyzes the first image for finding and determining features which are indicative of an orientation of the camera, allocating a distance of the camera to the real object, generating distance data indicative of the allocated distance, determining the pose of the camera with respect to a coordinate system related to the real object of the real environment using the distance data and the first orientation data. The method may be performed with reduced processing requirements and/or higher processing speed, in mobile device such as mobile phones having display, camera and orientation sensor.

US 9667860 B2 discloses a method, a computer readable medium and an apparatus for providing composition and position guidance for a camera. One method includes detecting an image received on a capturing device. The image is analyzed to identify objects and determine attributes of the objects. Suggestions are provided to adjust at least one of position or setting of the capturing device in accordance to rules, based on the analysis of the image. Adjustment of the capturing device based on the suggestions provided, are detected and used to receive an adjusted image. The adjusted image is captured by the capturing device.

Further, US 20110254860 A1 discloses a mobile device including a visual input device, for capturing external visual information having real visual background information, and a processing device. The processing device is for associating a selected application with the external visual information, and for executing the selected application based on the external visual information and on user-related input information. The processing device for generating a visual output signal related to at least one virtual visual object in response to the application is further configured to provide the visual output signal to a projector device included within the mobile device such that the projector device will be configured to project said visual output signal related to the at least one virtual visual object onto the visual background, thereby modifying said external visual information.

WO 2013103912 A1 discloses transaction visual capturing apparatuses, methods and systems ("TVC") transforming mobile device location coordinate information transmissions, real-time reality visual capturing, and mixed gesture capturing via TVC components into real-time behavior-sensitive product purchase related information, shopping purchase transaction notifications, and electronic receipts. In one implementation, the TVC obtains user check-in information from a user mobile device upon user entry into a store. The TVC extracts a user identifier based on the user check- in information, and accesses a database for a user profile. The TVC determines a user prior behavior pattern from the accessed user profile, and obtains user real-time in-store behavior data from the user mobile device.

US 2018/024049 A1 describes a method and a colorimetric device for performing colorimetric analysis of a test fluid to evaluate associated physiological parameters. The images of the test strip at different heights are captured by the calorimetric device and based on analysis of the captured images, a plurality of geometric parameters respectively associated with the test strip is determined. Based on the plurality of geometric parameters, an image resizing factor is determined and resized images are generated based on the image resizing factor. Upon generating the resized images, calorimetric values respectively associated with the resized images are determined based on which physiological parameters associated with the test fluid are evaluated.

EP 1 801 568 A1 describes a method involving positioning a camera at a test strip for pictorially detecting a color indicator and a reference color area. A measured value is determined for the relative position between the camera and the strip and compared with a desired value area. The camera is moved to reduce deflection relative to the strip during the deflection between the measured value and the desired value. An image area assigned to the indicator is localized in a colored image that is detected by the camera. An analyte concentration is determined in a sample by a comparison value.

US 2018/0024049 A1 discloses a method of performing colorimetric analysis of a test fluid to evaluate associated physiological parameters, said method comprising: receiving, by a processor of a colorimetric device, a first image of a test strip captured at a first height before dipping the test strip into the test fluid, and a second image of the test strip captured at a second height after dipping the test strip into the test fluid; determining, by the processor, a plurality of first and second geometric parameters respectively associated with the test strip based on analysis of the captured first and second images; deriving, by the processor, an image resizing factor based on the plurality of first and second geometric parameters and generating one of a resized first image and a resized second image based on the image resizing factor thus derived; determining, by the processor, first and second calorimetric values respectively associated with the resized first and the resized second images; and evaluating, by the processor, one or more physiological parameters associated with the test fluid based on change in luminosity determined based on the one or more predetermined calorimetric values, the one or more first and the second calorimetric values.

WO 2012/131386 A1 relates to capturing and processing assay test data using a mobile phone. Software may guide the user during image capture to assist the user in capturing a suitable image, e.g. correctly orienting the device, correctly focussing the device, and obtaining adequate illumination. One possible solution to simplify the processing, being to display a guide or template overlay showing the outline of the test strip and/or region of interest (or simply a rectangle of the correct proportions). If the image can be processed for suitability in near real time then the correct orientation may be indicated on the screen and image capture initiated automatically. One option for this interactive feedback being the change of colour of the template, overlay or guide marks, for example by changing from red (no suitable image) to green (suitable image), thus avoiding additional 'clutter' in the display. Similarly, the software may provide the user with an audio or tactile indication that an image has been acquired, e.g. by playing a simulated "camera shutter sound", a simple beep or activating an inbuilt vibration alert within the device.

EP 1 801 568 A1 relates to a method involving positioning a camera (8) at a test strip (1) for pictorially detecting a color indicator and a reference color area. A measured value is determined for the relative position between the camera and the strip and compared with a desired value area. The camera is moved to reduce deflection relative to the strip during the deflection between the measured value and the desired value. An image area assigned to the indicator is localized in a colored image that is detected by the camera. An analyte concentration is determined in a sample by a comparison value.

Despite the advantages involved in using mobile computing devices for the purpose of performing an analytical measurement, several technical challenges remain. Specifically, reliability and accuracy of the measurements need to be enhanced and ensured. A major challenge is the presence and impact of varying environmental conditions such as lighting conditions. Thus, measurement results may strongly be dependent on the environment to set up and/or background illumination and, thus, may vary from measurement to measurement, even under identical chemical or biochemical conditions. Furthermore, measurement results may depend on relative positioning of an illumination device and the camera of the mobile device which, due to a huge number of different mobile devices available on the market, may vary for different types or models of the mobile device. These technical challenges are even emphasized by the fact that, typically, when performing an analytical measurement by using optical test strips, at least two images need to be taken and analyzed, wherein one image shows at least part of a test field without having the sample applied thereto, whereas at least one second image is acquired having the sample applied thereto, wherein the application of the second image typically takes place after a certain time of waiting, until the color formation reaction has taken place. Since, in this case, at least two images need to be compared, wherein the test strip typically is handled and repositioned in between taking these two images the uncertainty of the measurement is additionally increased.

### Problem to be solved

It is therefore desirable to provide methods and devices which address the above-mentioned technical challenges of analytical measurements using mobile devices such as consumer-electronics mobile devices, specifically multipurpose mobile devices which are not dedicated to analytical measurements such as smart phones or tablet computers. Specifically, methods and devices shall be proposed which ensure reliability and accuracy of the measurements.

### Summary

This problem is addressed by methods, computer programs, computer-readable storage media and devices with the features of the independent claims. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, a method of performing an analytical measurement based on a color formation reaction in an optical test strip by using a mobile device having a camera is disclosed. The method comprises the following steps which, as an example, may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed.

In general, the method comprises the following steps:
a) providing an optical test strip having a test field without having a sample applied thereto;
b) capturing, by using the camera, at least one first image of at least part of the test field of the optical test strip without having a sample applied thereto;
c) applying a sample of bodily fluid to the test field of the optical test strip;
d) capturing, by using the camera, at least one second image of at least part of the test field of the optical test strip having the sample of bodily fluid applied thereto; and
e) determining an analytical measurement result value by using the first and the second image of the optical test field of the optical test strip,
f) wherein indication is provided by the mobile device where to take the second image, so that the first and second images are taken at similar local positions with respect to at least one environmental feature
   wherein the environmental feature comprises a tangible article, and wherein the environmental feature is different from the test strip, or parts of the test strip, and from the mobile device having a camera or parts thereof, and
   wherein the method comprises determining a first local position when taking the first image (214) and determining at least one second local position before or when taking the second image (222), wherein the method comprises comparing the first and second local positions in order to determine whether the first and second images (214, 222) are taken at similar local positions with respect to the at least one environmental feature.

Therein, specifically, method step f) may be performed before or during performing method step d), wherein the remaining method steps, specifically, may be performed in the given order. As outlined above, however, a different order, specifically for method steps a)-e), is also possible.

The term "analytical measurement" as used herein is a broad term and is to be given its ordinary and customary meaning to a person of ordinary skill in the art and is not to be limited to a special or customized meaning. The term specifically may refer, without limitation, to a quantitatively and/or qualitatively determination of at least one analyte in an arbitrary sample. The sample comprises a bodily fluid, such as blood, interstitial fluid, urine, saliva or other types of body fluids. The result of the analytical measurement is a concentration of the analyte and/or the presence or absence of the analyte to be determined. Specifically, as an example, the analytical measurement may be a blood glucose measurement, thus the result of the analytical measurement may for example be a blood glucose concentration. In particular, an analytical measurement result value may be determined by the analytical measurement. The term "analytical measurement result value" as used herein is a broad term and is to be given its ordinary and customary meaning to a person of ordinary skill in the art and is not to be limited to a special or customized meaning. The term specifically may refer, without limitation, to a numerical indication of an analyte concentration in a sample.

The at least one analyte, as an example, may be or may comprise one or more specific chemical compounds and/or other parameters. As an example, one or more analytes may be determined which take part in metabolism, such as blood glucose. Additionally or alternatively, other types of analytes or parameters may be determined, e.g. a pH value. The at least one sample, specifically, may be or may comprise at least one bodily fluid, such as blood, interstitial fluid, urine, saliva or the like. Additionally or alternatively, however, other types of samples may be used, such as water.

The analytical measurement is an analytical measurement including a change of at least one optical property of an optical test strip, which change ismeasured or determined visually by using the camera. Specifically, the analytical measurement comprises a color formation reaction in the presence of the at least one analyte to be determined. The term "color formation reaction" as used herein is a broad term and is to be given its ordinary and customary meaning to a person of ordinary skill in the art and is not to be limited to a special or customized meaning. The term specifically may refer, without limitation, to a chemical, biological or physical reaction during which a color, specifically a reflectance, of at least one element involved in the reaction, changes with the progress of the reaction.

The term "optical test strip" as used herein is a broad term and is to be given its ordinary and customary meaning to a person of ordinary skill in the art and is not to be limited to a special or customized meaning. The term specifically refers to an arbitrary element or device comprising at least one strip-shaped carrier, with the at least one test field applied thereto or integrated therein, the element being configured for performing a color-change detection reaction. The optical test strip may also be referred to as a test strip or a test element. The optical test strip has a test field containing at least one test chemical for detecting at least one analyte. The optical test strip comprises at least one substrate, such as at least one carrier, with the at least one test field applied thereto or integrated therein. In particular, the optical test strip may further comprise at least one white area, such as a white field, specifically in a proximity to the test field, for example enclosing or surrounding the test field. The white area may be a separate field independently arranged on the substrate or carrier. However, additionally or alternatively, the substrate or carrier itself may be or may comprise the white area. These test strips are generally widely in use and available. One test strip may carry a single test field or a plurality of test fields having identical or different test chemicals comprised therein.

As further used herein, the term "test field" is a broad term and is to be given its ordinary and customary meaning to a person of ordinary skill in the art and is not to be limited to a special or customized meaning. The term specifically may refer, without limitation, to a coherent amount of the test chemical, such as to a field, e.g. a field of round, polygonal or rectangular shape, having one or more layers of material, with at least one layer of the test field having the test chemical comprised therein.

The term "mobile device" as used herein is a broad term and is to be given its ordinary and customary meaning to a person of ordinary skill in the art and is not to be limited to a special or customized meaning. The term specifically may refer, without limitation, to a mobile electronics device, more specifically to a mobile communication device such as a cell phone or smartphone. Additionally or alternatively, as will be outlined in further detail below, the mobile device may also refer to a tablet computer or another type of portable computer having at least one camera.

The term "camera" as used herein is a broad term and is to be given its ordinary and customary meaning to a person of ordinary skill in the art and is not to be limited to a special or customized meaning. The term specifically may refer, without limitation, to a device having at least one imaging element configured for recording or capturing spatially resolved one-dimensional, two-dimensional or even three-dimensional optical data or information. As an example, the camera may comprise at least one camera chip, such as at least one CCD chip and/or at least one CMOS chip configured for recording images. As used herein, without limitation, the term "image" specifically may relate to data recorded by using a camera, such as a plurality of electronic readings from the imaging device, such as the pixels of the camera chip.

The camera, besides the at least one camera chip or imaging chip, may comprise further elements, such as one or more optical elements, e.g. one or more lenses. As an example, the camera may be a fix-focus camera, having at least one lens which is fixedly adjusted with respect to the camera. Alternatively, however, the camera may also comprise one or more variable lenses which may be adjusted, automatically or manually. The invention specifically shall be applicable to cameras as usually used in mobile applications such as notebook computers, tablets or, specifically, cell phones such as smart phones. Thus, specifically, the camera may be part of a mobile device which, besides the at least one camera, comprises one or more data processing devices such as one or more data processors. Other cameras, however, are feasible.

The camera specifically may be a color camera. Thus, such as for each pixel, color information may be provided or generated, such as color values for three colors R, G, B. a larger number of color values is also feasible, such as four color values for each pixel, for example R, G, G, B. Color cameras are generally known to the skilled person. Thus, as an example, the camera chip may consist of a plurality of three or more different color sensors each, such as color recording pixels like one pixel for red (R), one pixel for green (G) and one pixel for blue (B). For each of the pixels, such as for R, G, B, values may be recorded by the pixels, such as digital values in the range of 0 to 255, depending on the intensity of the respective color. Instead of using color triples such as R, G, B, as an example, quadruples may be used, such as R, G, G, B. The color sensitivities of the pixels may be generated by color filters or by appropriate intrinsic sensitivities of the sensor elements used in the camera pixels. These techniques are generally known to the skilled person.

Steps b) and d) each comprise capturing at least one image by using the camera. The term "capturing at least one image" may refer to one or more of imaging, image recording, image acquisition, image capturing. The term "capturing at least one image" may comprise capturing a single image and/or a plurality of images such as a sequence of images. For example, the capturing of the image may comprise recording continuously a sequence of images such as a video or a movie. The capturing of the at least one image may be initiated by the user action or may automatically be initiated, e.g. once the presence of the at least one object within a field of view and/or within a predetermined sector of the field of view of the camera is automatically detected. These automatic image acquisition techniques are known e.g. in the field of automatic barcode readers, such as from automatic barcode reading apps. The capturing of the images may take place, as an example, by acquiring a stream or "life stream" of images with the camera, wherein one or more of the images, automatically or by user interaction such as pushing a button, are stored and used as the at least one first image or the at least one second image, respectively. The image acquisition may be supported by a processor of the mobile device, and the storing of the images may take place in a data storage device of the mobile device.

In each of steps b) and d), at least one image of at least a part of the test field is captured, by using the camera. These images are referred to as "the at least one first image" and "the at least one second image", wherein the terms "first" and "second" are used for the purpose of nomenclature, only, without ranking or numbering these images and without giving any preferences. The term "of at least a part of the test field" or "of at least part of the test field" both refer to the fact that at least one part of the at least one test field should be visible in each of the images, wherein, in the first and second images, different parts of the at least one test field may be visible. Besides the at least one part of the test field, in each case, further parts of the optical test strip may be visible, such as at least one part of a substrate of the test strip.

In step b), the at least one first image is captured without having a sample applied to the test field. This at least one first image typically is also referred to as the "blank image", and, in typical evaluation methods, the image is used for reference purposes, in order to take into account variations of the color or other optical properties of the test field which are not due to the sample or the analyte itself. The sample application in step c) may take place, as an example, directly or indirectly, e.g. via at least one capillary element. The at least one second image, captured after sample application, is typically also referred to as the "wet image", even though the sample may have dried when the image is actually captured. The second image typically is taken after having waited for at least a predetermined waiting time, such as after five seconds or more, in order to allow for the detection reaction to take place.

Thus, as an example, the method may comprise, between steps c) and d), waiting for at least a predetermined minimum amount of time. This predetermined minimum amount of time specifically may be sufficient for a detection reaction to take place in the test strip. As an example, the minimum amount of waiting time may be at least 5 s.

In step e), an analytical measurement result value is determined by using the first and the second image of the optical test field of the optical test strip. The analytical measurement result value, as an example, may be a numerical value indicator of a result of the analytical measurement, such as indicative of the concentration of at least one analyte in the sample, such as a blood glucose concentration. For determining the analytical measurement result value from the at least one first image and the at least one second image, as an example, a predetermined or determinable relationship between information derived from the first and second images, such as color information or color change information, and the at least one analytical measurement result value may be used. This predetermined or determinable relationship, as an example, may be stored in a data storage device of the mobile device and/or in the processor of the mobile device. The processor, as an example, may be configured by software programming to derive at least one item of information from the first and second images, such as at least one color coordinate, and to apply the predetermined or determinable relationship to the at least one item of information. The correlation, as an example a transformation function, a transformation table or a lookup table, may be determined e.g. empirically and may, as an example, be stored in at least one data storage device of the mobile device, e.g. by the software, specifically by the app downloaded from an app store or the like. As an example for deriving the at least one item of information, the processor may be programmed in order to recognize, preferably automatically, e.g. by pattern recognition and/or other algorithms, the test field or the at least one part of the test field in the images. Thereof, the processor may be programmed for determining the at least one item of information, such as one or more color coordinates. The respective at least one item of information derived from the blank image may be used for normalizing, such as by dividing the at least one item of information derived from the wet image by the at least one item of information derived from the corresponding blank image or by subtracting the at least one item of information derived from the wet image from the at least one item of information derived from the blank image or vice a versa. Other ways of normalizing are feasible. The correlation, as an example a transformation function, a transformation table or a lookup table, may be determined e.g. empirically and may, as an example, be stored in at least one data storage device of the mobile device, e.g. by the software, specifically by the app downloaded from an app store or the like.

The method may further comprise the step of displaying the analytical measurement result value, such as on a display of the mobile device. Therein, one or more numerical values indicating the analytical measurement result value may be indicated, e.g. on a display. Additionally or alternatively, range indication regarding the analytical measurement result value may be indicated, such as by indicating whether the analytical measurement result value is within or without one or more predefined ranges. As an example, a displaying of ranges such as high range, target range or in low range may be performed, without necessarily indicating the numerical value of the analytical measurement result value. Thus, the display not necessarily needs to be the display of a numerical value. Other means of displaying the analytical measurement result value are feasible, wherein the display may be one or more of a visual display, an audible display or a haptic display. Additionally or alternatively, the method may comprise storing the analytical measurement result value in at least one data storage device of the mobile device. Again additionally and alternatively, the method may further comprise transmitting the analytical measurement result value via at least one interface and/or via at least one data transmission network, such as to another computer, e.g. for further evaluation.

As indicated above, the method is performed such that indication is provided by the mobile device where to take the second image, so that the first and second images are taken at similar local positions with respect to at least one environmental feature.

The environmental feature specifically is suited for defining a location and/or a coordinate system in space and/or which may be used as a location marker in the field of view of the camera. The environmental feature specifically isa feature which, between capturing the first and second images, is likely not to change position. The environmental feature may be or may comprise, e.g. a table on which the test strip is disposed during the measurement, or a part of the table, such as a surface structure of the table. The environmental feature comprises a tangible article . The environmental feature is different from the test strip, or parts of the test strip, and from the mobile device having a camera or parts thereof.

For detecting the at least one environmental feature, the method may comprise detecting the at least one environmental feature in one or both of the first and second images. For this purpose, as an example, the method may make use of image recognition, such as software-based automatic image recognition and/or image recognition by machine learning processes.

Consequently, the partial feature of feature f) indicating that the first and second images are taken at similar local positions with respect to at least one environmental feature may generally refer to the fact that the at least one environmental feature provides for a coordinate system defining the first and/or the second local positions. Thus, the local position of the taking of the first image may be detected and/or monitored with respect to the at least one environmental feature, or the local position of taking of the second image may be detected and/or monitored with respect to the at least one environmental feature, or both. Further exemplary embodiments will be given in detail below.

The providing of indication is generally denoted by a method step f), wherein method step f), may specifically be performed before or during performing method step d). In fact, method step f) may also be part of method step d), such as by capturing or recording a life image or an image stream with the camera of the mobile device and showing this image stream or life image on a display of the mobile device, wherein indication is provided by the mobile device where to take the second image, so that the first and second images are taken at similar local positions with respect to the at least one environmental feature. As an example and as will be outlined in further detail below, augmented reality may be used, such as by adding the indication regarding where to take the second image to the image stream or life image displayed on the display.

As will be outlined in further detail below, the term "local position" as used herein and as specifically used in the context of taking the first and second images, in the following also simply referred to as "position", is a broad term and is to be given its ordinary and customary meaning to a person of ordinary skill in the art and is not to be limited to a special or customized meaning. The term specifically may refer, without limitation, to at least one item of spatial information regarding one or more of the camera or the test field at the moment of capturing the image, such as when and/or during capturing the image, wherein the at least one spatial information takes into account the at least one environmental feature, such as by determining the position of the at least one environmental feature in a field of view of the camera. The at least one item of spatial information may refer to at least one of a spatial coordinate and/or a spatial orientation, such as at least one spatial coordinate and/or at least one spatial orientation in at least one coordinate system defined by the at least one environmental feature. The at least one item of spatial information may refer to at least one of an absolute item of spatial information or a relative item of spatial information, such as a relative item of spatial information relating to a spatial relation between the camera and the test field. Thus, as an example, an absolute item of spatial information may be determined for the camera or the test field in a coordinate system defined by the at least one environmental feature, and a relative item of spatial information may be determined for the other one of the camera of the test field. Alternatively, absolute items of spatial information may be determined for both the camera and the test field in the coordinate system defined by the at least one environmental feature.

As an example, the local positions may comprise at least one of: an absolute position of the camera in space with respect to the at least one environmental feature; an absolute position of the test field in space with respect to the at least one environmental feature; a relative position between the camera and the test field in a coordinate system defined by at least one environmental feature in a field of view of the camera; an orientation of the camera in space with respect to at least one environmental feature; an orientation of the test field in space with respect to at least one environmental feature; a relative angular orientation between the camera and the test field in a coordinate system defined by at least one environ-mental feature; a relative angular orientation between the camera and at least one environ-mental feature in a field of view of the camera; a relative angular orientation between the test field and at least one environmental feature in a field of view of the camera; a relative angular orientation between the camera and the test field in a coordinate system defined by at least one environmental feature in a field of view of the camera.

As will also be outlined in further exemplary detail below, the term "similar" as used herein and as specifically used in the context of the local positions of the first and second images is a broad term and is to be given its ordinary and customary meaning to a person of ordinary skill in the art and is not to be limited to a special or customized meaning. The term specifically may refer, without limitation, to the fact that the first and second images are taken at local positions which fulfill at least one predetermined or determinable similarity criterion. Thus, as an example, the local positions of the first and second images may be identical at least within a predetermined range of tolerance, such as a predetermined range of tolerance stored in at least one data storage device of the mobile device.

Thus, step f), i.e. the providing of the indication where to take the second image, so that the first and second images are taken at similar local positions with respect to the at least one environmental feature, may comprise providing at least one similarity criterion for the local positions when taking the first and second images. As an example, the similarity criterion may be provided by a data storage device of the mobile device, having stored therein the at least one similarity criterion.

The method may further comprise evaluating whether the similarity criterion is fulfilled. Thus, as an example, the processor may be configured for evaluating the local positions at which the first and second images are taken, wherein the position of the second image may be a current position in the process of capturing the second image, such as by taking an image sequence or life stream of images, and the processor may be configured for determining whether or not the at least one similarity criterion is fulfilled or not.

The at least one similarity criterion specifically comprises a local similarity criterion. The similarity of the positions refers to a similarity in terms of local similarity.

The evaluation whether the at least one similarity criterion is fulfilled specifically comprises comparing the local positions when taking the first and second images, specifically comparing the deviation between the local positions with at least one tolerance threshold. Thus, as an example, the processor may be configured for determining a deviation between the second local position and the first local position and may be configured for comparing the deviation with at least one tolerance threshold, such as at least one tolerance threshold stored in at least one data storage device of the mobile device.

The method comprises determining a first local position when taking the first image and determining at least one second local position before or when taking the second image. Therein, the first and second local positions are determined with respect to the at least one environmental feature, as outlined above, such as in at least one coordinate system defined by the at least one environmental feature. The method comprises comparing the first and second local positions in order to determine whether the first and second images are taken at similar local positions with respect to the at least one environmental feature. Thus, as outlined above, the processor is configured for determining the first and second local positions and comparing these first and second local positions and/or their deviation with at least one tolerance threshold, in order to determine whether the similarity criterion is fulfilled or not.

The indication provided in step f), i.e. the indication provided by the mobile device where to take the second image, so that the first and second images are taken at similar local positions with respect to the at least one environmental feature, specifically may comprise visual indication, more specifically visual indication on a display of the mobile device. The indication specifically may be provided at least partially as augmented reality on a display of the mobile device. The term "augmented reality" as used herein is a broad term and is to be given its ordinary and customary meaning to a person of ordinary skill in the art and is not to be limited to a special or customized meaning. The term specifically may refer, without limitation, to a method of overlaying, on a display, a current image, life image or image stream of a scene with one or more items of additional information, such as one or more visual indicators or the like. Thus, as an example, the augmented reality, in step f), may be provided by providing, on the display, one or more arrows, frames or lines indicating a preferred positioning of the camera and the test strip. Additionally or alternatively, text may be displayed, indicating in which direction the camera and/or the test strip may have to be moved. Other augmented reality is possible.

The method specifically may comprise determining, and optionally storing, at least one first item of location information regarding one or both of a position of the camera and a position of the test field when capturing the first image. Thus, the position of the camera and/or of the test field at the at least one point in time when the at least one first image is captured may be determined and optionally stored. For determining the position, various means are feasible which will be explained in further detail below. The at least one first item of local information specifically may comprise at least one of: an absolute position of the test field; an absolute position of the camera. Therein, the at least one first item of location information, such as one or more of the absolute positions listed before, may be determined with respect to the at least one environmental feature, such as in at least one coordinate system defined by the at least one environmental feature.

The determining of the at least one first item of location information may comprise image analysis of the at least one first image, in order to detect the at least one environmental feature in the at least one first image. Additionally or alternatively, however, other means of determining the at least one first item of location information may be applied. Thus, as an example, the at least one determining of the at least one first item of location information may comprise using at least one sensor of the mobile device, specifically at least one of a location sensor, a gyroscopic sensor, an optical sensor, an inclination sensor.

Thus, for determining the at least one first item of location information, one or more sensors of the mobile device itself may be used.

As outlined above, visual means such as image recognition means may be used for determining the at least one first item of location information. Thus, the determining of the at least one first item of location information may, as an alternative or in addition to using at least one sensor of the mobile device, comprise detecting the at least one environmental feature of a local environment.

The at least one first item of location information may comprise location information of one or both of the camera and the test field relative to the at least one environmental feature. Thus, as an example, the at least one environmental feature may be used by the processor for defining a coordinate system in space, wherein the location information of the camera and/or the test field may be defined in this coordinate system. Additionally or alternatively, the at least one first item of location information may simply refer to a spatial distance between one or more of the camera and the test field and the environmental feature.

The detecting of the at least one environmental feature specifically may comprise image recognition in at least one image provided by the camera, more specifically in a standstill image at the time the at least one first image is taken and/or in a life image and/or an image stream. Thus, as an example, the first image itself may be used and may be subjected to image recognition, thereby recognizing the at least one environmental feature. Thus, as an example, the processor may be configured for performing a histogram analysis of the image, such as of the first image, in order to determine a prominent environmental feature which may be used as a spatial reference.

The indication provided in step f), i.e. the indication provided by the mobile device where to take the second image, so that the first and second images are taken at similar local positions with respect to the at least one environmental feature, specifically may be provided on the basis of the first item of location information. Thus, the mobile device may be configured for recording the position of the camera and/or the test field when capturing the first image, specifically with respect to the at least one environmental feature, in order to give the user directions for where to capture the second image. The indication may comprise visual indication, specifically visual indication on a display of the mobile device, more specifically visual indication at least partially by using augmented reality on the display of the mobile device.

For being able to provide the indication in step f), the method may comprise determining, specifically live determining, and optionally storing in a data storage device of the mobile device, at least one second item of location information regarding one or both of a position of the camera and a position of the test field, and comparing the second item of location information with the first item of location information. The providing of the indication in step f), i.e. the indication where to take the second image, so that the first and second images are taken at similar local positions with respect to the at least one environmental feature, may comprise providing the indication such that the second item of location information when taking the second image is, at least within a predetermined range of tolerance, identical to the first item of location information.

Similar to the at least one first item of location information, the at least one second item of local information may comprise at least one of: an absolute position of the test field; an absolute position of the camera; a relative position between the camera and the test field, specifically at the point in time the second image is captured and/or before the second image is captured. Thus, the second item of location information specifically may be determined as an information stream before capturing the second image, in order to provide the indication continuously or repeatedly, so the user can adjust the position of the test field and/or the camera accordingly before capturing the second image. Again, the at least one second item of location information may be determined with respect to the at least one environmental feature, such as in at least one coordinate system defined by the at least one environ-mental feature.

For determining the at least one second item of location information, basically the same options as for determining the at least one first item of location information exist. Thus, as an example, at least one internal sensor and/or image recognition means may be used, as explained above with respect to the at least one first item of location information. Thus, the determining of the at least one second item of location information may comprise image analysis of the at least one second image, in order to detect the at least one environmental feature in the at least one second image. Additionally or alternatively, however, other means of determining the at least one second item of location information may be applied. Thus, as an example, the at least one determining of the at least one second item of location information may comprise using at least one sensor of the mobile device, specifically at least one of a location sensor, a gyroscopic sensor, an optical sensor, an inclination sensor. Additionally or alternatively, as outlined above, the determining of the at least one second item of location information may comprise detecting the at least one environmental feature of a local environment, such as the same environmental feature as used for determining the at least one first item of location information. The at least one second item of location information may comprise location information of one or both of the camera and the test field relative to the at least one environmental feature. The detecting of the at least one environmental feature may comprise image recognition in an image provided by the camera, more specifically in a life image. Thus, as outlined above, the at least one second item of location information may be determined continuously and/or repeatedly before and/or during capturing the at least one second image, in order to allow for the user to adjust the local position of one or both of the camera and the test field. As outlined above, the at least one environmental feature may comprise at least one of an article in the field of view of the camera or a structural feature of an article in the field of view of the camera. Other options are possible.

In case the at least one environmental feature is not in the field of view of the camera when preparing for taking the at least one second image, the mobile device may provide indication to the user, such as visual indication, specifically by augmented reality, on how to move and/or reorient the mobile device in order to bring the at least one environmental feature into the field of view. Thus, as an example, when taking the first image, the mobile device may determine the at least one first local position with respect to at least one environmental feature visible in a field of view of the camera. For taking the at least one second image, when the mobile phone detects that the environmental feature is not visible, the indication may be provided to the user indicating where to move and/or how to reorient the mobile device in order to bring the same at least one environmental feature into the field of view of the camera, again. Since, in this case, the environmental feature is, firstly, not available for determining the second local position, one or more internal and/or external sensors may be used for providing the indication. Thus, as an example, one or more internal or external sensors may be used for detecting if the mobile device, after taking the first image, has been moved and/or reoriented, in order to provide the indication, such as by indicating to the user that the mobile device has to be moved back and/or reoriented, in order to bring the environmental feature into the field of view. Once the at least one environmental feature is in the field of view, as an example, image analysis may be used for determining the second local position with respect to the at least one environmental feature.

The capturing of the at least one second image may be one or both of user-initiated or automatically initiated. Specifically, the capturing may be automatically initiated as soon as the mobile device recognizes that the condition of the second image being captured at a similar local position as the first image before is fulfilled. Thus, step d) may be initiated automatically when at least one of the camera and the test field are in a predetermined position, such that the first and second images are taken at similar local positions with respect to the at least one environmental feature.

For capturing the at least one first image, basically two options may be considered. Thus, the first image may be captured basically at an arbitrary and non-predefined local position. As outlined above, the local position when capturing the first image may be determined and/or stored in a data storage device. The capturing of the first image may, as an example, be user-initiated, e.g. as soon as the user has placed the test strip in a suitable place, such as on a table. As a second option, the local position of one or both the test field and the camera when capturing the at least one first image may be determined by the mobile device, wherein indication may be provided to the user similar to step f) regarding where to take the first image. For possible options of this indication, reference may be made to the description of step f). Thus, before performing step b), indication may be provided by the mobile device where to take the first image. As an example, the mobile device may determine a suitable environmental feature in an image such as in an image stream and/or in a life image before capturing the first image, and, when a suitable feature is detected, may propose a position close to that feature to the user by providing corresponding indication. The indication comprises visual indication, specifically visual indication on a display of the mobile device, more specifically visual indication at least partially by using augmented reality on the display of the mobile device.

The above-mentioned options may also be used as possible alternatives, at the user's discretion. Thus, as an example, the user may override the proposal provided by the mobile device and may use a different local setup for capturing the first image.

The mobile device may further be configured for providing guidance to the user on one or more of the procedural steps. Thus, as an example, instructions may be provided on the display to perform one or more actions. As an example, user guidance for one or more of steps a) to d) may be provided in a visual format and/or by other means, such as audio guidance.

As an example, in the sample application in step c), user guidance may be provided. Thus, step c) may comprise at least one of:
- the mobile device prompting a user to apply the sample, specifically a drop, of bodily fluid to the test field of the optical test strip;
- the mobile device prompting the user to confirm application of the sample of bodily fluid to the test field of the optical test strip.

The prompting, as outlined above, may take place by the mobile device providing corresponding guidance to the user, in a visual format and/or by other means, such as acoustic means. As an example, user guidance may be provided on the display.

Similarly, user guidance may be provided by the mobile device for performing step a). Thus, step a) may comprise at least one of:
- the mobile device prompting a user to provide the optical test strip having the test field without having the sample applied thereto;
- the mobile device prompting the user to confirm having provided the optical test strip having the test field without having the sample applied thereto;
- the mobile device automatically detecting, specifically by image recognition, whether the optical test strip has been provided.

For the options of prompting, reference may be made to the description given above. Thus, even the steps of providing the optical test strip and of applying the sample to the test strip may at least partially be supported by the mobile device.

As outlined above, the method in one or more of the embodiments disclosed may fully or partially be computer-implemented. Thus, in a further aspect, a computer program is proposed comprising instructions which, when the program is executed by a mobile device having a camera, specifically by a processor of the mobile device, cause the mobile device to carry out the method as described herein, more specifically at least steps b), d), e) and f) of the method, wherein also steps a) and c) may, at least be partially computer-implemented or at least computer-supported.

The computer program specifically may be designed as an App. The App, as an example, may be downloaded from a download server.

As generally used therein, a "computer" may refer to a device having at least one processor and optionally further elements, such as one or more interfaces, one or more data storage devices, one or more user interfaces and the like. The term "processor" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math coprocessor or a numeric coprocessor, a plurality of registers, specifically registers configured for supplying operands to the ALU and storing results of operations, and a memory, such as an L1 and L2 cache memory. In particular, the processor may be a multi-core processor. Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like.

The computer program may further comprise instructions which, when the program is executed by the mobile device, may further prompt the user to perform steps a) and/or c) or to confirm having performed steps a) and/or c). For the possible options, reference may be made to the description of the method above.

In a further aspect, a computer-readable storage medium is disclosed, specifically a non-transitory storage medium, comprising instructions which, when executed by a mobile device having a camera, specifically by a processor of the mobile device, cause the mobile device to carry out the method according to the present invention, such as according to any one of the embodiments disclosed above and/or according to any one of the embodiments disclosed in further detail below. Specifically, as outlined above, at least steps b), d), e) and f) may be performed, wherein also one or both of steps a) and c) may at least partially be computer-implemented or at least computer-supported, as outlined above. Thus, as discussed above, the computer-readable storage medium may further comprise instructions which, when executed by the mobile device, further prompt a user to perform steps a) and/or c) or to confirm having performed steps a) and/or c).

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

The computer program may also be embodied as a computer program product. As used herein, a computer program product may refer to the program as a tradable product. The product may generally exist in an arbitrary format, such as in a paper format, or on a computer-readable data carrier and/or on a computer-readable storage medium. Specifically, the computer program product may be distributed over a data network.

In a further aspect, a mobile device for performing an analytical measurement is disclosed. For definitions and options of the mobile device, reference may be made to the description of the method given above. The mobile device comprises at least one camera and may comprise one or more processors. The mobile device is configured for performing at least steps b), d), e) and f) of the method of performing an analytical measurement according to the present invention, such as according to any one of the embodiments disclosed above and/or according to any one of the embodiments disclosed in further detail below. Thus, the processor of the mobile device may be software-configured for performing and/or controlling the execution of the method, at least of steps b), d), e) and f) of the method, wherein also steps a) and/or c) may at least partially be controlled and/or supported by the processor, as outlined above.

The mobile device may comprise further elements. Thus, as an example, the mobile device may further comprise at least one illumination source configured for illuminating the test field. As an example, the mobile device may comprise at least one light-emitting diode. The processor may also be configured for controlling the illumination source, such as during image capturing steps b) and/or d).

As outlined above, the mobile device may comprise at least one processor being programmed for controlling at least one of steps b), d), e), and f). For definitions and options regarding the design of the processor, reference may be made to the description given above.

In a further aspect, a kit for performing an analytical measurement is disclosed. The kit comprises:
- at least one mobile device according to any one of the preceding claims referring to a mobile device; and
- at least one optical test strip having at least one test field.

The term "kit" as used herein is a broad term and is to be given its ordinary and customary meaning to a person of ordinary skill in the art and is not to be limited to a special or customized meaning. The term specifically may refer, without limitation, to an assembly of a plurality of components, wherein the components each may function and may be handled independently from each other, wherein the components of the kit may interact to perform a common function.

The invention in any one of the aspects described herein may provide for a large number of advantages over known methods and devices of this kind. Thus, specifically, the invention may address the technical challenges referred to above. As outlined above, smartphone-based methods typically require capturing at least two images, wherein at least one image is taken before sample application and at least one thereafter, which may be referred to as the "wet" or final image. The present invention may allow for using augmented reality for improvement of the positioning during taking the first and second images. Thus, the same or at least similar positioning of the test strip during capturing the first or blank image and the at least one second or final image acquisition after sample application may be allowed.

Generally, the invention may greatly improve measurement performance of analytical measurements. Thus, the measurement performance of smartphone-based optical analysis of test strips may typically strongly depend on the conditions under which the images before and after sample application are taken. Ideally, the conditions are the same for both images. Augmented reality can be used to guide a user in order to take both images under very similar conditions. For positioning, position sensors of the mobile device phone and/or image recognition techniques may be used to determine the conditions, in order to improve measurement performance.

Therein, several options exist. Thus, a predefined position for capturing both the blank image and the final image may be used. Additionally or alternatively, the blank image may be captured at basically an arbitrary first local position, wherein the mobile device may determine and/or mark the first local position, and may later on use the first local position in order to provide user guidance such that the user is directed to the correct position for taking the second image.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

### In the Figures:

- Figure 1: shows a perspective view of a mobile device for use in an embodiment of the method of performing an analytical measurement;
- Figure 2: shows a flowchart of an embodiment of a method of performing an analytical measurement;
- Figures 3 and 4: exemplarily show first and second images taken before and after sample application; and
- Figure 5: shows comparative blood glucose measurements.

### Detailed description of the embodiments

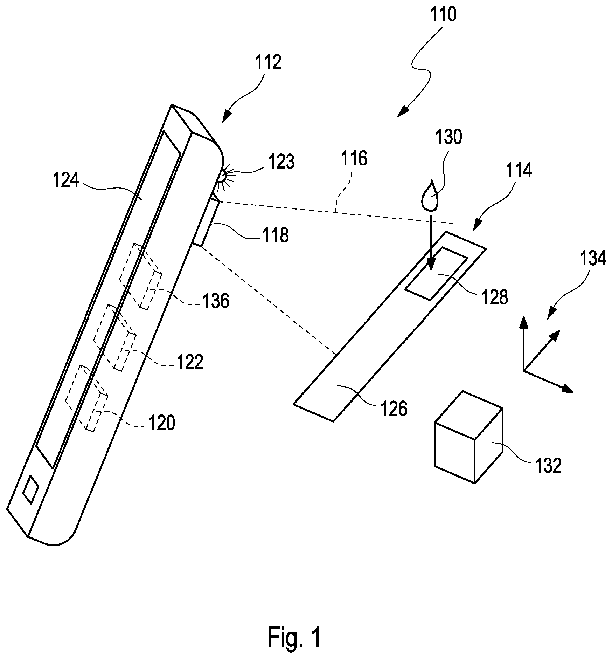

In Figure 1, an exemplary kit 110 for performing an analytical measurement is shown in a perspective view. The kit 110 comprises a mobile device 112 such as a smart phone and, further, at least one optical test strip 114, in this setup placed in a field of view 116 of a camera 118 of the mobile device 112.

The mobile device 112 may comprise, besides the at least one camera 118, at least one processor 120 and at least one data storage device 122. The mobile device 112 may further comprise at least one display 124, such as for displaying a life image taken by the camera 118 and/or for displaying information to a user. The mobile device 112 may further comprise at least one illumination source 123, such as an LED or the like.

The optical test strip 114 may comprise at least one substrate 126 such as a flexible, strip-shaped substrate. The optical test strip 114 further comprises at least one test field 128 applied to the substrate, the test field 128 comprising at least one test chemical for performing a detection reaction with at least one analyte comprised by a sample 130. The sample 130 may directly or indirectly be applied to the test field 128, such as by applying a droplet of a bodily fluid to the test field 128 and/or to a capillary element for conducting the sample 130 to the test field 128.

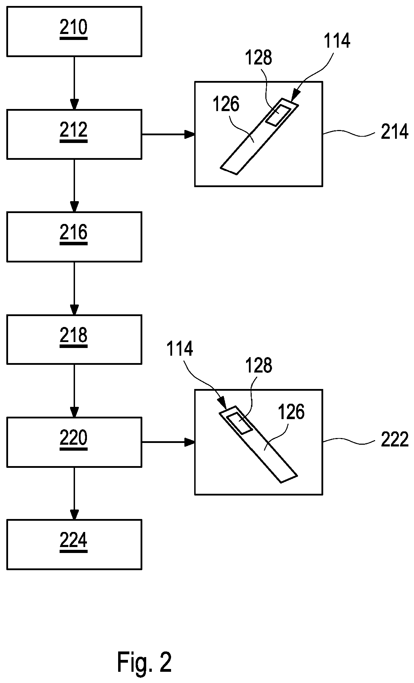

The mobile device 112 is configured, by appropriate programming of the processor 120, for use in the method according to the present invention which will be described with reference to an exemplary embodiment shown in a flow chart in Figure 2.

In a first step (step a)), denoted by reference number 210, the optical test strip 114 is provided. A user, as an example, may take the optical test strip from a test strip container. Step 210 may be supported by the mobile device 112, such as by prompting the user to provide the optical test strip 114, e.g. by displaying a corresponding message on the display 124. Additionally or alternatively, the mobile device 112 may also prompt the user to confirm that the optical test strip 114 has been provided, e.g. in the field of view 116 of the camera. Again additionally or alternatively, the mobile device 112 may also be configured for automatically detecting, e.g. by image recognition means, that the optical test strip 114 has been provided.

In a further step (step b)), denoted by reference number 212, the mobile device 112 captures, by using the camera 118, at least one first image 214 of at least part of the test field 128 of the optical test strip 114 without having the sample 130 applied thereto, as symbolically depicted in Figure 2. The method comprises determining a first local position when taking the first image 214. The first image 214 may be stored in the data storage device 122 of the mobile device 112. The first image 214 may also be referred to as a "blank image", and the first image 214 may comprise a single image or a series of images such as an image stream. The capturing of the first image 214 may be triggered by the user and/or may fully or partially be supported by the mobile device 112, e.g. by automatically triggering the image capture once the optical test strip 114 and/or the test field 128 have been recognized. For capturing the first image 214, the optical test strip 114 may be placed, by the user, in an arbitrary position relative to the camera 118 and/or relative to at least one environmental feature 132 in the field of view 116, which is symbolically depicted in Figure 1. In this case, the mobile device 112 specifically may be configured for determining a first local position when capturing the first image 214, and, optionally, storing the first local position in the data storage device 122. Additionally or alternatively, the mobile device 112 may also be configured for providing user guidance on where to capture the first image 214, by indicating e.g. an appropriate position on the display 124.

In a further step (step c)), denoted by reference number 216, the sample 130 of the bodily fluid is directly or indirectly applied to the test field 128 of the optical test strip 114. Again, as in step 210, this sample application may optionally be supported by the mobile device 112, such as by prompting the user to apply the sample 130 and/or by prompting the user to confirm that the sample 130 has been applied. Again, as an example, corresponding instructions may be displayed on the display 124. Additionally or alternatively, the mobile device 112 may also be configured for automatically detecting sample application.

After sample application in step 216, the method may provide for an appropriate waiting time. As an example, the waiting time may be at least 5 seconds, in order to allow for the detection reaction to take place. The waiting time may be displayed on the display 124, and/or the mobile device 112 may be configured for preventing further steps until the waiting time has expired.

In a further step (step f)), denoted by reference number 218, the mobile device 112 provides indication to the user where to take or capture a second image of at least part of the test field 128 of the optical test strip 114 having the sample 130 of bodily fluid applied thereto, so that the first and second images are taken at similar local positions with respect to the at least one environmental feature 132 wherein the environmental feature comprises a tangible article, and wherein the environmental feature is different from the test strip, or parts of the test strip, and from the mobile device having a camera or parts thereof. Therein, as an example, the local positions may be determined by using a coordinate system 134 defined by the at least one environmental feature 132 in Figure 1. The local positions may comprise one or more of absolute positions of the optical test strip 114, the camera 118 or both, or relative positions. The term "position", as outlined above, may also include orientations, such as angular orientations. For performing step f), the mobile device 112 may, as an example, record and/or display a life stream of images of the field of view 116 and may provide, also on the display 124, augmented reality with instructions for the user on where to position one or both of the test field 128 and the camera 118. Exemplary embodiments will be shown below.

In a further step (step d)), denoted by reference number 220 in Figure 2, the at least one second image, denoted by reference number 222, of at least part of the test field 128 of the optical test strip 114 is captured, having the sample 130 of bodily fluid applied thereto. When taking the second image 222 (or before) at least one second local position the method comprises determining at least one second local position. wherein the method further comprises comparing the first and second local positions in order to determine whether the first and second images 214, 222 are taken at similar local positions with respect to the at least one environmental feature. Again, this capturing may be triggered automatically, such as when the mobile device 112 recognizes that the camera 118 and/or the test field 128 are positioned correctly, such that, at least within a predetermined range of tolerance, the first and second images 214, 222 are taken at identical positions, i.e. at similar local positions with respect to the at least one environmental feature 132 fulfilling one or more predetermined similarity criteria.

In a further step (step e)), denoted by reference number 224, the mobile device 112 is configured for determining at least one analytical measurement result value by using the first and second images 214 and 222 of the optical test field 128 of the optical test strip 114. Therein, one or more evaluation algorithms may be applied to the images 214 and 222 by the processor 120. As an example, the processor 120 may derive at least one color coordinate from each of the images 214 and 222, in order to determine a color change between the blank image 214 and the final image 222 induced by the detection reaction. From the color change, e.g. by using a predetermined correlation or calibration function or a lookup table, the analytical measurement result value may be determined, such as a concentration of at least one analyte of interest. As an example, a concentration of glucose in blood or interstitial fluid may be determined as the analytical measurement result value. Other possibilities exist.

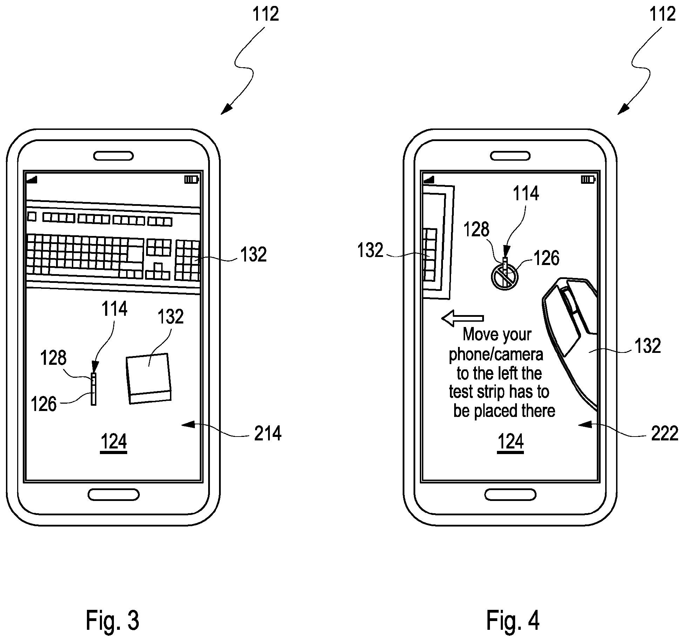

In Figures 3 and 4, live images displayed on the display 124 of the mobile device 112 are shown, including augmented reality. Therein, as an example, Figure 3 shows the situation when capturing the first image 214, wherein Figure 4 shows the live image before capturing the second image 222. As can be seen, a plurality of environmental features 132 is detected in the field of view 116 of the camera 118, such as a computer keyboard on a table, a cube, a computer mouse or simply a pattern of the table, such as a wooden texture. These environmental features 132 may be used, as in Figure 1, for determining local positions of the test field 128 and/or the camera 118, e.g. by image recognition methods. Additionally or alternatively, one or more sensors 136 of the mobile device 112 may be used for determining the first and/or second local positions.

As can be seen in Figure 4, augmented reality may be used for providing indication where to take the second image 222, so that the first and second images 214, 222 are taken at similar local positions with respect to the at least one environmental feature 132. The augmented reality may comprise symbols, such as the "no parking" sign and the arrow, as well as text, such as text comprising instructions on the required positional corrections of the test field 128 and/or the camera 118.

Generally, as outlined above, two options may be considered. Thus, as a first option, the first image 214 may be captured at an arbitrary first local position. The mobile device 112 may be configured for determining the first local position and store the first local position, such as in the data storage device 122. When capturing the second image 222, indication is provided by the mobile device 112, e.g. by the augmented reality shown in Figure 4, where to take the second image 222, so that the first and second images 214, 222 are taken at similar local positions with respect to the at least one environmental feature 132. As a second option, the mobile device 112 may provide indication already where to capture the first image 214, e.g. by the augmented reality shown in Figure 4, and, similarly, also for the capturing of the second image 222.

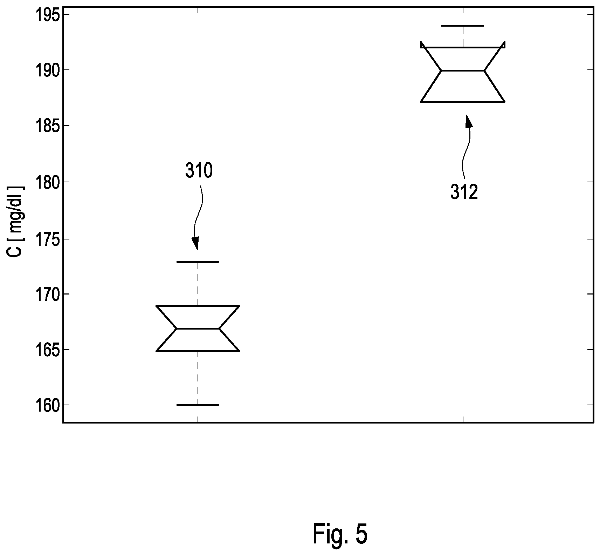

In Figure 5, measurement results are shown which demonstrate the effect of controlling the local positions for capturing the blank image and the final image. For these experiments, blood glucose measurements were performed using an optical test strip 114 and a sample 130. Two different setups were used: In a first setup, denoted by reference number 310, the blank images or first images 214 and the final images or second images 222 were taken at identical local positions. In a second setup, denoted by reference number 312, the blank images or first images 214 were taken at a common first local position, and the final images or second images 222 were taken at a common second local position, wherein the second local position differed from the first local position. In each setup, 10 measurements were performed, wherein for the blank images a fresh optical test strip 114 was used (no sample applied), whereas for the final images an optical test strip 114 was used 3 days after sample application for demonstration purposes (the test field of this strip had constant optical properties different from a fresh optical test strip).

The measurement results are shown in Figure 5. Therein, on the horizontal axis, the two different setups 310, 312 are shown. On the vertical axis, the determined analytical measurement result is shown, in this case a blood glucose concentration c in mg/dl. The results are shown as box plots for both setups 310, 312. As can be seen, a significant difference occurs between the correct or controlled setup 310 and the uncontrolled setup 312. The difference is supposed to be mainly due to differing illumination conditions in the first and second local positions. However, other influences may also occur. The difference clearly shows the benefit of the present invention, since taking the first and second images 214, 222 at similar local positions with respect to the at least one environmental feature provides for increasing the reproducibility of the measurements.

### List of reference numbers

- 110: kit for performing an analytical measurement
- 112: mobile device
- 114: optical test strip
- 116: field of view
- 118: camera
- 120: processor
- 122: data storage device
- 123: illumination source
- 124: display
- 126: substrate
- 128: test field
- 130: sample
- 132: environmental feature
- 134: coordinate system
- 136: sensor
- 210: providing optical test strip
- 212: capturing first image of test field without having a sample applied thereto
- 214: first image
- 216: applying sample of bodily fluid to the test field of the optical test strip
- 218: providing indication by the mobile device where to take the second image, so that the first and second images are taken at similar local positions with respect to the at least one environmental feature
- 220: capturing second image of test field having the sample of bodily fluid applied thereto
- 222: second image
- 224: determining an analytical measurement result value
- 310: first setup: blank image and final image taken at identical local positions
- 312: second setup: blank image and final image taken at different local positions

## Claims

1. A method of performing an analytical measurement based on a color formation reaction in an optical test strip (114) by using a mobile device (112) having a camera (118), the method comprising:
a) providing an optical test strip (114) having a test field (128) without having a sample (130) of bodily fluid applied thereto;
b) capturing, by using the camera (118), at least one first image (214) of at least part of the test field (128) of the optical test strip (114) without having a sample (130) of bodily fluid applied thereto;
c) applying a sample (130) of bodily fluid to the test field (128) of the optical test strip (114);
d) capturing, by using the camera (118), at least one second image (222) of at least part of the test field (128) of the optical test strip (114) having the sample (130) applied thereto; and
e) determining an analytical measurement result value by using the first image (214) and the second image (222) of the optical test field (128) of the optical test strip (114),
f) wherein indication is provided by the mobile device (112) where to take the second image (222), so that the first and second images (214, 222) are taken at similar local positions with respect to at least one environmental feature (132), wherein the environmental feature (132) is a feature which, between capturing the first (214) and second (222) images, has a fixed and/or unchanged position, wherein the environmental feature comprises a tangible article, and wherein the environmental feature is different from the test strip, or parts of the test strip, and from the mobile device having a camera or parts thereof, and
wherein the method comprises determining a first local position when taking the first image (214) and determining at least one second local position before or when taking the second image (222), wherein the method comprises comparing the first and second local positions in order to determine whether the first and second images (214, 222) are taken at similar local positions with respect to the at least one environmental feature.

2. The method according to the preceding claim, wherein the method further comprises, between steps c) and d), waiting for at least a predetermined minimum amount of time.

3. The method according to any one of the preceding claims, wherein the local positions comprise at least one of: an absolute position of the camera (118) in space with respect to the at least one environmental feature (132); an absolute position of the test field (128) in space with respect to the at least one environmental feature (132); a relative position between the camera (118) and the test field (128) in a coordinate system defined by the at least one environmental feature (132) in a field of view of the camera (118); an orientation of the camera (118) in space with respect to the at least one environmental feature (132); an orientation of the test field (128) in space with respect to the at least one environmental feature (132);a relative angular orientation between the camera (118) and the at least one environmental feature (132) in a field of view of the camera (118); a relative angular orientation between the test field (128) and the at least one environmental feature (132) in a field of view of the camera (118); a relative angular orientation between the camera (118) and the test field (128) in a coordinate system defined by the at least one environmental feature (132) in a field of view of the camera (118).

4. The method according to any one of the preceding claims, wherein the indication provided by the mobile device (112) where to take the second image (222), so that the first and second images (214, 222) are taken at similar local positions with respect to the at least one environmental feature, is provided at least partially as augmented reality on a display of the mobile device (112).

5. The method according to any one of the preceding claims, wherein the method comprises determining at least one first item of location information regarding one or both of a position of the camera (118) and a position of the test field (128) when capturing the first image (214).

6. The method according to the preceding claim, wherein the determining of the at least one first item of location information comprises at least one of:
- using at least one sensor (136) of the mobile device (112);
- detecting the at least one environmental feature (132) of a local environment, wherein the at least one first item of location information comprises location information of one or both of the camera (118) and the test field (128) relative to the at least one environmental feature (132).

7. The method according to any one of the two preceding claims, wherein the method comprises determining at least one second item of location information regarding one or both of a position of the camera (118) and a position of the test field (128), and comparing the second item of location information with the first item of location information, wherein the providing of the indication where to take the second image (222), so that the first and second images (214, 222) are taken at similar local positions with respect to the at least one environmental feature, comprises providing the indication such that the second item of location information when taking the second image (222) is, at least within a predetermined range of tolerance, identical to the first item of location information.

8. The method according to any one of the preceding claims, wherein the method further comprises, before performing step b), providing indication by the mobile device (112) where to take the first image (214).

9. The method according to any one of the preceding claims, wherein step c) comprises at least one of:
- the mobile device (112) prompting a user to apply the sample (130) of bodily fluid to the test field (128) of the optical test strip (114);
- the mobile device (112) prompting the user to confirm application of the sample (130) of bodily fluid to the test field (128) of the optical test strip (114).

10. The method according to any one of the preceding claims, wherein the environmental feature (132) comprises an article or a part thereof.

11. The method according to any one of the preceding claims, wherein step a) comprises at least one of:
- the mobile device (112) prompting a user to provide the optical test strip (114) having the test field (128) without having the sample (130) applied thereto;
- the mobile device (112) prompting the user to confirm having provided the optical test strip (114) having the test field (128) without having the sample (130) applied thereto;
- the mobile device (112) automatically detecting whether the optical test strip (114) has been provided.

12. A computer program comprising instructions which, when the program is executed by a mobile device (112) having a camera (118), cause the mobile device (112) to carry out at least steps b), d), e) and f) of the method according to any one of the preceding claims.

13. A computer-readable storage medium comprising instructions which, when executed by a mobile device (112) having a camera (118) cause the mobile device (112) to carry out at least steps b), d), e) and f) of the method according to any one of the preceding method claims.

14. A mobile device (112) for performing an analytical measurement, the mobile device (112) having at least one camera (118), the mobile device (112) being configured for performing at least steps b), d), e) and f) of the method of performing an analytical measurement according to any one of the preceding claims referring to a method of performing an analytical measurement.

15. A kit (110) for performing an analytical measurement, the kit (110) comprising:
- at least one mobile device (112) according to any one of the preceding claims referring to a mobile device (112); and
- at least one optical test strip (114) having at least one test field (128).

## Patentansprüche

1. Verfahren zur Durchführung einer analytischen Messung basierend auf einer Farbbildungsreaktion in einem optischen Teststreifen (114) unter Verwendung einer mobilen Vorrichtung (112) mit einer Kamera (118), wobei das Verfahren Folgendes umfasst:
a) Bereitstellen eines optischen Teststreifens (114) mit einem Testfeld (128), auf dem keine Probe (130) von Körperflüssigkeit aufgegeben ist;
b) Aufnehmen, unter Verwendung der Kamera (118), mindestens eines ersten Bildes (214) von mindestens einem Teil des Testfeldes (128) des optischen Teststreifens (114), auf dem keine Probe (130) von Körperflüssigkeit aufgegeben ist;
c) Aufgeben einer Probe (130) von Körperflüssigkeit auf das Testfeld (128) des optischen Teststreifens (114);
d) Aufnehmen, unter Verwendung der Kamera (118), mindestens eines zweiten Bildes (222) von mindestens einem Teil des Testfeldes (128) des optischen Teststreifens (114), auf dem die Probe (130) aufgegeben ist; und
e) Bestimmen eines analytischen Messergebniswerts unter Verwendung des ersten Bildes (214) und des zweiten Bildes (222) des optischen Testfeldes (128) des optischen Teststreifens (114),
f) wobei eine Angabe durch die mobile Vorrichtung (112) bereitgestellt wird, wo das zweite Bild (222) aufzunehmen ist, sodass das erste und das zweite Bild (214, 222) an ähnlichen lokalen Positionen in Bezug auf mindestens ein Umgebungsmerkmal (132) aufgenommen werden, wobei das Umgebungsmerkmal (132) ein Merkmal ist, das zwischen dem Aufnehmen des ersten (214) und des zweiten (222) Bildes eine feste und/oder unveränderte Position aufweist,
wobei das Umgebungsmerkmal einen greifbaren Gegenstand umfasst und wobei sich das Umgebungsmerkmal von dem Teststreifen oder Teilen des Teststreifens und von der mobilen Vorrichtung mit einer Kamera oder Teilen davon unterscheidet, und
wobei das Verfahren das Bestimmen einer ersten lokalen Position beim Aufnehmen des ersten Bildes (214) und das Bestimmen mindestens einer zweiten lokalen Position vor oder beim Aufnehmen des zweiten Bildes (222) umfasst, wobei das Verfahren das Vergleichen der ersten und der zweiten lokalen Position umfasst, um zu bestimmen, ob das erste und das zweite Bild (214, 222) an ähnlichen lokalen Positionen in Bezug auf das mindestens eine Umgebungsmerkmal aufgenommen werden.

2. Verfahren nach dem vorhergehenden Anspruch, wobei das Verfahren ferner zwischen den Schritten c) und d) das Warten für mindestens eine vorbestimmte Mindestzeitdauer umfasst.

3. Verfahren nach einem der vorhergehenden Ansprüche, wobei die lokalen Positionen mindestens eines umfassen von: einer absoluten räumlichen Position der Kamera (118) in Bezug auf das mindestens eine Umgebungsmerkmal (132); einer absoluten räumlichen Position des Testfeldes (128) in Bezug auf das mindestens eine Umgebungsmerkmal (132); einer relativen Position zwischen der Kamera (118) und dem Testfeld (128) in einem Koordinatensystem, das durch das mindestens eine Umgebungsmerkmal (132) in einem Sichtfeld der Kamera (118) definiert ist; einer räumlichen Ausrichtung der Kamera (118) in Bezug auf das mindestens eine Umgebungsmerkmal (132); einer räumlichen Ausrichtung des Testfeldes (128) in Bezug auf das mindestens eine Umgebungsmerkmal (132); einer relativen Winkelausrichtung zwischen der Kamera (118) und dem mindestens einen Umgebungsmerkmal (132) in einem Sichtfeld der Kamera (118); einer relativen Winkelausrichtung zwischen dem Testfeld (128) und dem mindestens einen Umgebungsmerkmal (132) in einem Sichtfeld der Kamera (118); einer relativen Winkelausrichtung zwischen der Kamera (118) und dem Testfeld (128) in einem Koordinatensystem, das durch das mindestens eine Umgebungsmerkmal (132) in einem Sichtfeld der Kamera (118) definiert ist.

4. Verfahren nach einem der vorhergehenden Ansprüche, wobei die durch die mobile Vorrichtung (112) bereitgestellte Angabe, wo das zweite Bild (222) aufzunehmen ist, sodass das erste und das zweite Bild (214, 222) an ähnlichen lokalen Positionen in Bezug auf das mindestens eine Umgebungsmerkmal aufgenommen werden, mindestens teilweise als erweiterte Realität auf einer Anzeige der mobilen Vorrichtung (112) bereitgestellt wird.

5. Verfahren nach einem der vorhergehenden Ansprüche, wobei das Verfahren das Bestimmen mindestens eines ersten Elements von Standortinformation in Bezug auf eine oder beide von einer Position der Kamera (118) und einer Position des Testfeldes (128) beim Aufnehmen des ersten Bildes (214) umfasst.

6. Verfahren nach dem vorhergehenden Anspruch, wobei das Bestimmen des mindestens einen ersten Elements von Standortinformation mindestens eines umfasst von:
- Verwenden mindestens eines Sensors (136) der mobilen Vorrichtung (112);
- Nachweisen des mindestens einen Umgebungsmerkmals (132) einer lokalen Umgebung, wobei das mindestens eine erste Element von Standortinformation Standortinformationen von einem oder beiden von der Kamera (118) und dem Testfeld (128) relativ zu dem mindestens einen Umgebungsmerkmal (132) umfasst.

7. Verfahren nach einem der zwei vorhergehenden Ansprüche, wobei das Verfahren das Bestimmen mindestens eines zweiten Elements von Standortinformation in Bezug auf eine oder beide von einer Position der Kamera (118) und einer Position des Testfeldes (128) und das Vergleichen des zweiten Elements von Standortinformation mit dem ersten Element von Standortinformation umfasst, wobei das Bereitstellen der Angabe, wo das zweite Bild (222) aufzunehmen ist, sodass das erste und das zweite Bild (214, 222) an ähnlichen lokalen Positionen in Bezug auf das mindestens eine Umgebungsmerkmal aufgenommen werden, das Bereitstellen der Angabe derart umfasst, dass das zweite Element von Standortinformation beim Aufnehmen des zweiten Bildes (222) mindestens innerhalb eines vorbestimmten Toleranzbereichs mit dem ersten Element von Standortinformation identisch ist.

8. Verfahren nach einem der vorhergehenden Ansprüche, wobei das Verfahren ferner vor dem Durchführen von Schritt b) das Bereitstellen einer Angabe durch die mobile Vorrichtung (112) umfasst, wo das erste Bild (214) aufzunehmen ist.

9. Verfahren nach einem der vorhergehenden Ansprüche, wobei Schritt c) mindestens eines umfasst von:
- Auffordern eines Benutzers durch die mobile Vorrichtung (112), die Probe (130) von Körperflüssigkeit auf das Testfeld (128) des optischen Teststreifens (114) aufzugeben;
- Auffordern des Benutzers durch die mobile Vorrichtung (112), das Aufgeben der Probe (130) von Körperflüssigkeit auf das Testfeld (128) des optischen Teststreifens (114) zu bestätigen.

10. Verfahren nach einem der vorhergehenden Ansprüche, wobei das Umgebungsmerkmal (132) einen Gegenstand oder einen Teil davon umfasst.

11. Verfahren nach einem der vorhergehenden Ansprüche, wobei Schritt a) mindestens eines umfasst von:
- Auffordern eines Benutzers durch die mobile Vorrichtung (112), den optischen Teststreifen (114) mit dem Testfeld (128) bereitzustellen, auf dem keine Probe (130) aufgegeben ist;
- Auffordern des Benutzers durch die mobile Vorrichtung (112), das Bereitstellen des optischen Teststreifens (114) mit dem Testfeld (128), auf dem keine Probe (130) aufgegeben ist, zu bestätigen;
- automatisches Nachweisen durch die mobile Vorrichtung (112), ob der optische Teststreifen (114) bereitgestellt wurde.

12. Computerprogramm, umfassend Anweisungen, die, wenn das Programm von einer mobilen Vorrichtung (112) mit einer Kamera (118) ausgeführt wird, die mobile Vorrichtung (112) veranlassen, mindestens die Schritte b), d), e) und f) des Verfahrens nach einem der vorhergehenden Ansprüche auszuführen.

13. Computerlesbares Speichermedium, umfassend Anweisungen, die, wenn sie von einer mobilen Vorrichtung (112) mit einer Kamera (118) ausgeführt werden, die mobile Vorrichtung (112) veranlassen, mindestens die Schritte b), d), e) und f) des Verfahrens nach einem der vorhergehenden Verfahrensansprüche auszuführen.

14. Mobile Vorrichtung (112) zur Durchführung einer analytischen Messung, wobei die mobile Vorrichtung (112) mindestens eine Kamera (118) aufweist, wobei die mobile Vorrichtung (112) zur Durchführung mindestens der Schritte b), d), e) und f) des Verfahrens zur Durchführung einer analytischen Messung nach einem der vorhergehenden Ansprüche, die sich auf ein Verfahren zur Durchführung einer analytischen Messung beziehen, konfiguriert ist.

15. Kit (110) zur Durchführung einer analytischen Messung, wobei der Kit (110) Folgendes umfasst:
- mindestens eine mobile Vorrichtung (112) nach einem der vorhergehenden Ansprüche, die sich auf eine mobile Vorrichtung (112) beziehen; und
- mindestens einen optischen Teststreifen (114) mit mindestens einem Testfeld (128).

## Revendications

1. Procédé de réalisation d'une mesure analytique basée sur une réaction de formation de couleur dans une bandelette de test optique (114) en utilisant un dispositif mobile (112) ayant une caméra (118), le procédé comprenant :
a) la fourniture d'une bandelette de test optique (114) ayant un champ de test (128) sans l'application d'un échantillon (130) de fluide corporel sur celui-ci ;
b) la capture, en utilisant la caméra (118), d'au moins une première image (214) d'au moins une partie du champ de test (128) de la bandelette de test optique (114) sans l'application d'un échantillon (130) de fluide corporel sur celui-ci ;
c) l'application d'un échantillon (130) de fluide corporel sur le champ de test (128) de la bandelette de test optique (114) ;
d) la capture, en utilisant la caméra (118), d'au moins une deuxième image (222) d'au moins une partie du champ de test (128) de la bandelette de test optique (114) sur lequel est appliqué l'échantillon (130) ; et
e) la détermination d'une valeur de résultat de mesure analytique en utilisant la première image (214) et la deuxième image (222) du champ de test optique (128) de la bandelette de test optique (114),
f) dans lequel une indication est fournie par le dispositif mobile (112) sur où prendre la deuxième image (222), de sorte que les première et deuxième images (214, 222) sont prises au niveau de positions locales similaires par rapport à au moins une caractéristique environnementale (132), dans lequel la caractéristique environnementale (132) est une caractéristique qui, entre la capture des première (214) et deuxième (222) images, a une position fixe et/ou inchangée,
dans lequel la caractéristique environnementale comprend un article tangible, et dans lequel la caractéristique environnementale est différente de la bandelette de test, ou de parties de la bandelette de test, et du dispositif mobile ayant une caméra ou de parties de celui-ci, et
dans lequel le procédé comprend la détermination d'une première position locale lors de la prise de la première image (214) et la détermination d'au moins une deuxième position locale avant ou lors de la prise de la deuxième image (222), dans lequel le procédé comprend la comparaison des première et deuxième positions locales afin de déterminer si les première et deuxième images (214, 222) sont prises au niveau de positions locales similaires par rapport à l'au moins une caractéristique environnementale.

2. Procédé selon la revendication précédente, dans lequel le procédé comprend en outre, entre les étapes c) et d), l'attente pendant au moins une durée minimale prédéterminée.

3. Procédé selon l'une quelconque des revendications précédentes, dans lequel les positions locales comprennent au moins l'une parmi : une position absolue de la caméra (118) dans l'espace par rapport à l'au moins une caractéristique environnementale (132) ; une position absolue du champ de test (128) dans l'espace par rapport à l'au moins une caractéristique environnementale (132) ; une position relative entre la caméra (118) et le champ de test (128) dans un système de coordonnées défini par l'au moins une caractéristique environnementale (132) dans un champ de vision de la caméra (118) ; une orientation de la caméra (118) dans l'espace par rapport à l'au moins une caractéristique environnementale (132) ; une orientation du champ de test (128) dans l'espace par rapport à l'au moins une caractéristique environnementale (132) ; une orientation angulaire relative entre la caméra (118) et l'au moins une caractéristique environnementale (132) dans un champ de vision de la caméra (118) ; une orientation angulaire relative entre le champ de test (128) et l'au moins une caractéristique environnementale (132) dans un champ de vision de la caméra (118) ; une orientation angulaire relative entre la caméra (118) et le champ de test (128) dans un système de coordonnées défini par l'au moins une caractéristique environnementale (132) dans un champ de vision de la caméra (118).

4. Procédé selon l'une quelconque des revendications précédentes, dans lequel l'indication fournie par le dispositif mobile (112) sur où prendre la deuxième image (222), de sorte que les première et deuxième images (214, 222) sont prises au niveau de positions locales similaires par rapport à l'au moins une caractéristique environnementale, est fournie au moins en partie en tant que réalité augmentée sur un écran du dispositif mobile (112).

5. Procédé selon l'une quelconque des revendications précédentes, dans lequel le procédé comprend la détermination d'au moins un premier élément d'informations d'emplacement concernant une ou les deux parmi une position de la caméra (118) et une position du champ de test (128) lors de la capture de la première image (214).

6. Procédé selon la revendication précédente, dans lequel la détermination de l'au moins un premier élément d'informations d'emplacement comprend au moins l'une parmi :
- l'utilisation d'au moins un capteur (136) du dispositif mobile (112) ;
- la détection de l'au moins une caractéristique environnementale (132) d'un environnement local, dans lequel l'au moins un premier élément d'informations d'emplacement comprend des informations d'emplacement d'un ou les deux parmi la caméra (118) et le champ de test (128) par rapport à l'au moins une caractéristique environnementale (132).

7. Procédé selon l'une quelconque des deux revendications précédentes, dans lequel le procédé comprend la détermination d'au moins un deuxième élément d'informations d'emplacement concernant une ou les deux parmi une position de la caméra (118) et une position du champ de test (128), et la comparaison du deuxième élément d'informations d'emplacement avec le premier élément d'informations d'emplacement, dans lequel la fourniture de l'indication sur où prendre la deuxième image (222), de sorte que les première et deuxième images (214, 222) sont prises au niveau de positions locales similaires par rapport à l'au moins une caractéristique environnementale, comprend la fourniture de l'indication de telle sorte que le deuxième élément d'informations d'emplacement lors de la prise de la deuxième image (222) est, au moins dans une plage de tolérance prédéterminée, identique au premier élément d'informations d'emplacement.

8. Procédé selon l'une quelconque des revendications précédentes, dans lequel le procédé comprend en outre, avant la réalisation de l'étape b), la fourniture d'une indication par le dispositif mobile (112) sur où prendre la première image (214).

9. Procédé selon l'une quelconque des revendications précédentes, dans lequel l'étape c) comprend au moins l'un parmi :
- le dispositif mobile (112) invitant un utilisateur à appliquer l'échantillon (130) de fluide corporel sur le champ de test (128) de la bandelette de test optique (114) ;
- le dispositif mobile (112) invitant l'utilisateur à confirmer l'application de l'échantillon (130) de fluide corporel sur le champ de test (128) de la bandelette de test optique (114).

10. Procédé selon l'une quelconque des revendications précédentes, dans lequel la caractéristique environnementale (132) comprend un article ou une partie de celui-ci.

11. Procédé selon l'une quelconque des revendications précédentes, dans lequel l'étape a) comprend au moins l'un parmi :
- le dispositif mobile (112) invitant un utilisateur à fournir la bandelette de test optique (114) ayant le champ de test (128) sans l'application de l'échantillon (130) sur celui-ci ;
- le dispositif mobile (112) invitant l'utilisateur à confirmer avoir fourni la bandelette de test optique (114) ayant le champ de test (128) sans l'application de l'échantillon (130) sur celui-ci ;
- le dispositif mobile (112) détectant automatiquement si la bandelette de test optique (114) a été fournie.

12. Programme informatique comprenant des instructions qui, lorsque le programme est exécuté par un dispositif mobile (112) ayant une caméra (118), amènent le dispositif mobile (112) à effectuer au moins les étapes b), d), e) et f) du procédé selon l'une quelconque des revendications précédentes.

13. Support de stockage lisible par ordinateur comprenant des instructions qui, lorsqu'elles sont exécutées par un dispositif mobile (112) ayant une caméra (118), amènent le dispositif mobile (112) à effectuer au moins les étapes b), d), e) et f) du procédé selon l'une quelconque des revendications de procédé précédentes.

14. Dispositif mobile (112) permettant la réalisation d'une mesure analytique, le dispositif mobile (112) ayant au moins une caméra (118), le dispositif mobile (112) étant configuré pour réaliser au moins les étapes b), d), e) et f) du procédé de réalisation d'une mesure analytique selon l'une quelconque des revendications précédentes se référant à un procédé de réalisation d'une mesure analytique.

15. Kit (110) permettant la réalisation d'une mesure analytique, le kit (110) comprenant :
- au moins un dispositif mobile (112) selon l'une quelconque des revendications précédentes se référant à un dispositif mobile (112) ; et
- au moins une bandelette de test optique (114) ayant au moins un champ de test (128).
